# EUROPEAN PATENT SPECIFICATION

(11) **EP 4 157 180 B1**
(45) Date of publication and mention of the grant of the patent: **11.06.2025**
(21) Application number: 20937378.6
(22) Date of filing: 27.05.2020
(51) Int. Cl.: A61F 13/15, A61F 13/511, A61F 13/51

(54) **ABSORBENT ARTICLE WITH TOPSHEET COMPRISING CELLULOSE-BASED FIBERS**
ABSORBIERENDER ARTIKEL MIT DECKSCHICHT AUS ZELLULOSEHALTIGEN FASERN
ARTICLE ABSORBANT AVEC FEUILLE SUPÉRIEURE COMPRENANT DES FIBRES À BASE DE CELLULOSE

(43) Date of publication of application: 05.04.2023
(73) Proprietor: The Procter & Gamble Company, Cincinnati, OH 45202 (US)
(72) Inventor: LIU, Zhe, Beijing 101312 (CN); YAO, Lu, Beijing 101312 (CN)
(74) Representative: P&G Patent Germany
(86) International application number: PCT/CN2020/092579
(87) International publication number: WO 2021/237507

(56) References cited:
- WO-A1-2013/191077
- WO-A1-2014/171388
- WO-A1-2017/104512
- CN-A- 1 406 566
- CN-A- 107 920 939
- JP-A- 2005 324 010
- US-A1- 2019 117 473

## Description

### FIELD OF THE INVENTION

The present invention relates to an absorbent article comprising a topsheet which comprises nonwoven comprising cellulose-based fibers.

### BACKGROUND OF THE INVENTION

Nonwovens are widely used as components as a topsheet in a variety of absorbent articles for personal hygiene, such as disposable diapers for infants, training pants for toddlers, adult incontinence undergarments and/or sanitary napkins which are designed to absorb and contain body exudates, in particular large quantities of urine, runny bowel movement (BM) and/or menses.

Topsheets in absorbent articles are expected to penetrate bodily fluids quickly, and prevent fluid flow-back.

Nonwovens may comprise a variety of fiber types, including, for example, synthetic fibers such as polyethylene, polypropylene, and mixtures thereof. Recently, there has been an increased interest in supplementing or substituting synthetic components of nonwovens with naturally-derived components or cellulose-based fibers. For example, natural fibers especially natural cellulose-based fibers may be mixed with synthetic fibers, or may take the place of synthetic fibers, in nonwovens. Absorbent articles comprising nonwovens comprising natural fibers may be perceived by consumers as being of higher quality, more environmentally friendly, and/or softer against the skin of the wearer when utilized in a topsheet, an outer cover, or other context. Cellulose-based fibers such as cotton, for example, may exhibit an increased tendency to form hydrogen bonds with polar fluids as compared to synthetic fibers. When polar fluids, such as urine and menses contact with and/or enter a nonwoven topsheet comprising cellulose-based fibers, the polar fluids instead of passing through the nonwoven topsheet and into an acquisition layer and/or an absorbent core underneath the topsheet, may form hydrogen bonds with at least some of the cellulose-based fibers, thus swelling the cellulose-based fibers and trapping fluid in or on the nonwoven topsheet.

Capillary forces relied on to draw fluids from the nonwoven topsheet and into the acquisition layer and/or absorbent core may not be strong enough to overcome the hydrogen bonds formed between the polar fluids and cellulose-based fibers of the nonwoven topsheet when a nonwoven containing cellulose-based fibers is used as a topsheet in absorbent articles. This swelling of the cellulose-based fibers may be undesirable because the polar fluids may remain in and/or on the topsheet and proximate to and/or in contact with the skin of the wearer, which is not consumer desired.

Nonwoven topsheets may be chemically treated to make the topsheet hydrophobic, more hydrophobic, or less hydrophilic, and thus at least inhibit polar fluids from bonding with the cellulose-based fibers of the nonwoven topsheet. Modification of a surface of nonwoven hydrophobic by for example applying a hydrophobic treatment composition may reduce hydrogen bonds between the cellulose-based fibers and polar fluids but it also brings a challenge for liquid penetration. Especially, when the fluid is in a small amount such as the end part of a continuous urine flow, there is insufficient gravity to drive the fluid to overcome the hydrophobicity to strike through the nonwoven. As a result, it would smear on the surface of the topsheet to cause skin wetness. Nonwoven topsheets may be chemically treated to alter contact angles of one or both surfaces of the nonwoven topsheet. WO2014/171388 discloses nonwoven which has hydrophilic surfaces and contact angles decreasing in a z-direction. WO2017/104512 discloses a laminate nonwoven comprising a first fiber layer having a contract angle of 75-100° and a second fiber layer having a contact angle less than 75°. While these treatment processes may result in a topsheet that reduces or prevents polar fluids from bonding with the natural fibers of the topsheet, the resulting topsheet may also have significantly increased rewet resulting in topsheets that may leave the skin wet, which is not desired.

US2019117473 relates to a topsheet for use in an absorbent article. The topsheet has at least a first layer. The first layer comprises at least 15% by weight of natural fibers by total weight of the first layer. The first layer has a plurality of apertures. The first layer comprises land areas between the plurality of the apertures. The contact angle on the land areas of the first layer between the plurality of the apertures is more than 70°, according to the Contact Angle Test Method. The topsheet has a run-off of less than 40%, according to the Run-off Test Method.

WO2017104512 discloses a layered non-woven cloth having two adjacent fiber layers, wherein one of the two fiber layers has higher hydrophilicity than the other fiber layer, and at least one of the fiber layers includes a liquid film-splitting agent.

### SUMMARY OF THE INVENTION

The present invention relates to an absorbent article comprising a wearer-facing surface and an opposite garment-facing surface, the absorbent article further comprising a liquid pervious topsheet, a liquid impervious backsheet, and an absorbent core disposed between the topsheet and the backsheet, wherein the topsheet comprises a nonwoven substrate comprising cellulose-based fibers, a first surface and an opposite second surface, the first surface of the nonwoven substrate forming at least part of the wearer-facing surface,
wherein the first surface comprises a first area having a first contact angle no less than about 100°, as measured by Contact Angle Test, and the second surface comprises a second area having a second contact angle no less than about 82°, as measured by Contact Angle Test, the second contact angle being smaller than the first contact angle, and wherein a difference between the first contact angle and the second contact angle is in the range of about 15° to about 45°; and wherein the first surface comprises a first treatment composition, and wherein the second surface comprises a second treatment composition which is the same as the first treatment composition.

For ease of discussion, the apertured nonwoven and the absorbent article will be discussed with reference to the numerals referred to in these figures. The figures and detailed description should however not be considered limiting the scope of the claims, unless explicitly indicated otherwise, and the invention disclosed herein is also used in a wide variety of absorbent article forms.

### BRIEF DESCRIPTION OF THE DRAWINGS

In the drawings, like numerals or other designations designate like features throughout the views.
Fig. 1 is a schematic plan view of an exemplary absorbent article according to the present invention.
Fig. 2 is a lateral cross-section view along 2-2 of the absorbent article of Fig. 1.
Fig. 3 is a schematic cross-sectional view of a nonwoven substrate according to the present invention.
Fig. 4 is a schematic illustration of an apparatus for applying a treatment composition to a nonwoven substrate.
Fig. 5 is a schematic illustration of another apparatus for applying a treatment composition to a nonwoven substrate.
Fig. 6 is a schematic illustration of another apparatus for applying a treatment composition to a nonwoven substrate.
Fig. 7 is a schematic illustration of a weight used in Rewet Test.

### DETAILED DESCRIPTION OF THE INVENTION

Various non-limiting forms of the present disclosure will now be described to provide an overall understanding of the principles of the structure, function, manufacture, and use of an absorbent article comprising back ears having unique engineering strain properties and low surface roughness. One or more examples of these non-limiting embodiments are illustrated in the accompanying drawings. Those ordinary skilled in the art will understand that the absorbent articles described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

"Absorbent article" refers to wearable devices, which absorb and/or contain liquid, and more specifically, refers to devices, which are placed against or in proximity to the body of the wearer to absorb and contain the various exudates discharged from the body. Absorbent articles can include diapers, training pants, adult incontinence undergarments, feminine hygiene products such as sanitary napkins and pantyliners, and wipes.

As used herein, the term "comprising" means that the various components, ingredients, or steps can be conjointly employed in practicing the present invention. Accordingly, the term "comprising" is open-ended and encompasses the more restrictive terms "consisting essentially of" and "consisting of".

As used herein, the term "cellulose-based fibers" intends to include both natural cellulose fibers such as pulp and cotton, and regenerated cellulose fibers such as rayon (including viscose, lyocell, MODAL (a product of Lenzing AG, Lenzing, Austria) and cuprammonium rayon) unless specified differently.

As used herein, the terms "hydrophilic" and "hydrophobic" have meanings that are well established in the art with respect to the contact angle of water on the surface of a material. Thus, a material having a water contact angle of greater than about 90° is considered hydrophobic, and a material having a water contact angle of less than about 90° is considered hydrophilic.

As used herein, the term "natural fibers" refers to elongated substances produced by plants and animals and comprises animal-based fibers and plant-based fibers. Natural fibers may comprise fibers harvested without any post-harvest treatment step as well as those having a posttreatment step, such as, for example, washing, scouring, and bleaching.

As used herein, the term "plant-based fibers" comprises both harvested fibers and synthetic fibers that comprise bio-based content. Harvested plant-based fibers may comprise cellulosic matter, such as wood pulp; seed hairs, such as cotton; stem (or bast) fibers, such as flax and hemp; leaf fibers, such as sisal; and husk fibers, such as coconut.

### Absorbent Article

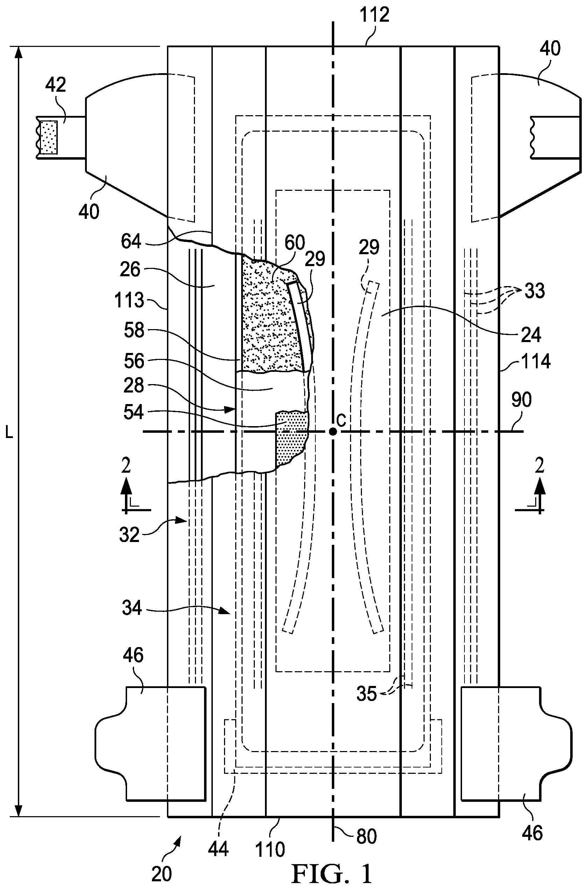

Absorbent articles will now be generally discussed and further illustrated in the form of a baby diaper 20 as exemplarily represented in Fig. 1. Fig. 1 is a plan view of the exemplary diaper 20 in a flattened-out configuration with the taped ends opened and the garment-facing side turned up. An article that is presented to the user closed such as a training pant may also be represented flattened out by cutting it along its side waists. The absorbent article will typically have a front edge 110, a back edge 112 and the longitudinally-extending lateral side edges 113, 114. The front edge 110 forms the edge of the front waist and the back edge 112 of the back waist, which together when worn by the wearer form the opening for the waist of the wearer. The lateral edges 113, 114 can each form one of the leg openings. The absorbent article 20 notionally comprises a longitudinal centerline 80 dividing the article in a left side and a right side, and a perpendicular transversal centerline 90 disposed at half the length of the article as measured on the longitudinal centerline 80, with both centerlines crossing at the center point C. The taped back ends 42 attached on the front of the diaper to such as a landing zone 44.

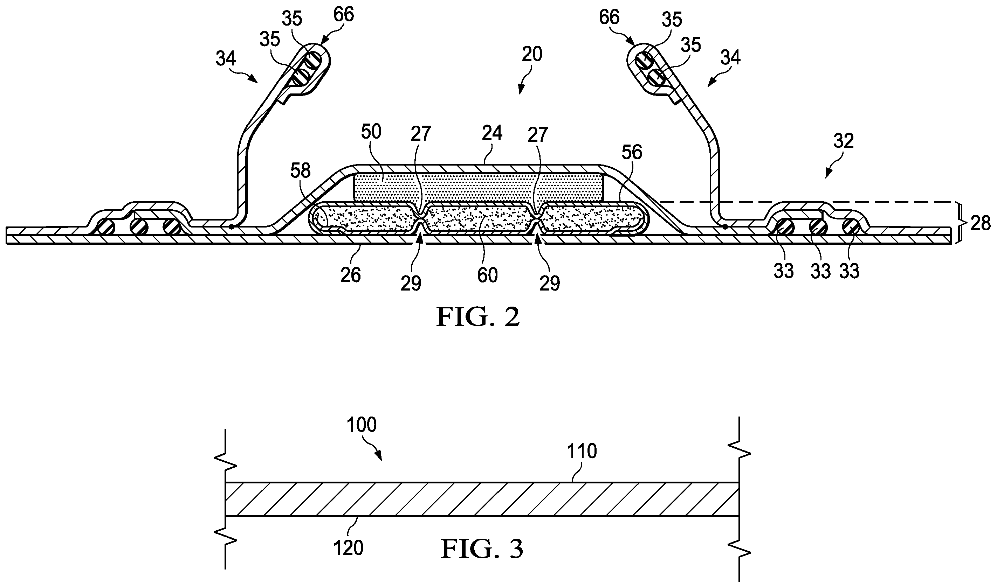

Other layers of the absorbent article are better illustrated in Fig. 2, which shows in cross-section in addition to the liquid permeable topsheet 24 and the backsheet 26, an absorbent core 28 between the topsheet 24 and the backsheet 26.

An optional acquisition and/or distribution layer (or system) 50 is represented in Fig. 2 together with other typical diaper components. The acquisition and/or distribution layer may comprise one layer or more than one layer. Typical acquisition and/or distribution layers may not comprise SAP as this may slow the acquisition and distribution of the fluid, but an additional layer may also comprise SAP if some fluid retention properties are wished.
The absorbent article may typically comprise a pair of partially upstanding barrier leg cuffs 34 having elastic elements 35 and elasticized gasketing cuffs 32 having elastic elements 33 substantially planar with the chassis. Both types of cuffs are typically joined to the chassis of the absorbent article typically via bonding to the topsheet and/or backsheet.
The absorbent article may comprise elasticized back ears 40 having a tape end 42 which can be attached to a landing zone 44 at the front of the article, and front ears 46 typically present in such taped diapers.

### Topsheet

Referring to Figs. 1 and 2, the topsheet 24 is a part of the absorbent article 10 that is in contact with the wearer's skin. The topsheet 24 may be joined to portions of the backsheet 26, the absorbent core 28, the barrier leg cuffs 34, and/or any other layers as is known to those of ordinary skill in the art. The topsheet 24 may be compliant, soft-feeling, and non-irritating to the wearer's skin. Further, at least a portion of, or all of, the topsheet 24 may be liquid permeable, permitting liquid bodily exudates to readily penetrate through its thickness.

The topsheet may comprise one layer or more than one layer. The topsheet may comprise a plurality of three dimensional elements such as protrusion, recesses, apertures and any combination thereof, so that the topsheet has a three-dimensional structure.

In an absorbent article according to the present invention, the topsheet comprises a nonwoven substrate disclosed herein. The nonwoven substrates suitable for use as the topsheet comprises cellulose-based fibers.

The topsheet may comprise between about 20% and about 100%, between about 50% and about 100%, or between about 65% and 100%, by weight of the nonwoven topsheet of cellulose-based fibers. In one embodiment, the topsheet may comprise about 50% cellulose-based fibers and about 50% synthetic fibers. In another example, the topsheet may comprise about 20% cellulose-based fibers and about 80% synthetic fibers. In yet another example, the topsheet may comprise about 100% cellulose-based fibers, such as about 100% cotton fibers.

The topsheet may comprise a second layer positioned underneath the nonwoven substrate. The second layer may comprise synthetic fibers.

The topsheet may have a rewet lower than about 70mg, or lower than about 65mg, or lower than about 60mg, as measured by Rewet Test disclosed herein.

Further details regarding the nonwoven substrate of the present disclosure used as topsheets are discussed in dedicated sections further below.

### Absorbent core

As used herein, the term "absorbent core" refers to a component used or intended to be used in an absorbent article and which comprises an absorbent material and optionally a core wrap. As used herein, the term "absorbent core" does not include the topsheet, the backsheet and any acquisition-distribution layer or multilayer system, which is not integral part of the absorbent core. The absorbent core is typically the component of an absorbent article that has the most absorbent capacity of all the components of the absorbent article. The terms "absorbent core" and "core" are herein used interchangeably.

Referring to Figs. 1 and 2, the absorbent core 28 can absorb and contain liquid received by the absorbent article and comprise an absorbent material 60, which may be cellulose fibers, a blend of superabsorbent polymers and cellulose fibers, pure superabsorbent polymers, and/or a high internal phase emulsion foam. The absorbent core 28 may comprise absorbent material free channels 29, through which the top side 56 of the core wrap may be bonded to the bottom side 58 of the core wrap. The core wrap bonds 27 may at least persist as the absorbent core 28 swells upon liquid absorption and creates three-dimensional channels at the wearer-facing surface of the article. Of course, this is entirely optional, the absorbent core may also not have bonded channels, or even unbonded channels. The absorbent material defines an absorbent material area 8, which may be rectangular as show in in Fig. 1, but it is also common to have a shaped area which is tapered in the area around the transversal centerline 90.

The absorbent material comprises a liquid-absorbent material commonly used in disposable absorbent articles such as comminuted wood pulp, which is generally referred to as airfelt or fluff. Examples of other suitable liquid-absorbent materials include creped cellulose wadding; melt blown polymers, including co-form; chemically stiffened, modified or cross-linked cellulosic fibers; tissue, including tissue wraps and tissue laminates, absorbent foams, absorbent sponges, superabsorbent polymers (herein abbreviated as "SAP"), absorbent gelling materials, or any other known absorbent material or combinations of materials.
The absorbent material in the absorbent core can be any type. It can be an airfelt core comprising wood cellulose fibers such as pulp fibers mixed with SAP, or an airfelt-free core free from such cellulose fibers. Airfelt cores typically comprises from 40% to 80% of SAP. For absorbent cores comprising a relatively high proportion of SAP at least partially enclosed within the core wrap, the SAP content may represent in particular at least 80%, 85%, 90%, 95% and up to 100%, of superabsorbent polymer by weight of the absorbent material. The absorbent material may in particular comprise no or only small amount of cellulose fibers, such as less than 20%, in particular less than 10%, 5% or even 0% of cellulose fibers by weight of the absorbent material. The absorbent core may comprise an absorbent material comprising at least 80%, at least 90%, at least 95%, or at least 99% by weight of the absorbent core. The term "superabsorbent polymer" refers herein to absorbent material, which may be cross-linked polymer, and that can typically absorb at least 10 times their weight of an aqueous 0.9% saline solution as measured using the Centrifuge Retention Capacity (CRC) test (EDANA method WSP 241.2-05E). The SAP may in particular have a CRC value of more than 20 g/g, or more than 24 g/g, or of from 20 to 50 g/g, or from 20 to 40 g/g, or from 24 to 30 g/g. The SAP may be typically in particulate forms (superabsorbent polymer particles), but it not excluded that other forms of SAP may be used such as a superabsorbent polymer foam for example.

### Nonwoven Substrate

The present invention provides a nonwoven substrate suitable for a topsheet of an absorbent article. A nonwoven substrate 100 disclosed herein, referring to Fig. 3, comprises a first surface 110 and a second surface 120. The first surface 110 comprises a first area having a first contact angle no less than about 100°, or no less than about 105°, no less than about 108°, as measured by Contact Angle Test disclosed herein. The first surface 110 of the nonwoven substrate 100 may comprise the first area at least about 60%, or at least about 70%, or at least about 80% of the first surface 110. The first surface 110 of the nonwoven substrate 100 may consist of the first area.

The second surface 120 comprises a second area having a second contact angle no less than about 82°, or no less than about 84°, as measured by Contact Angle Test disclosed herein. The second contact angle is smaller than the first contact angle. The second surface 120 of the nonwoven substrate 100 may comprise the second area at least about 60%, or at least about 70%, or at least about 80% of the second surface 120. The second surface 120 of the nonwoven substrate 100 may consist of the second area.

A difference between the first contact angle and the second contact angle is in the range of about 15° to about 45°, or in the range of about 15° to about 40°, or in the range of about 20° to about 45°.

In some embodiments, the first surface may have a first contact angle no greater than about 145°.

Natural cellulose-based fibers, such as, for example, cotton fibers, may be naturally hydrophobic due to the presence of naturally occurring waxy and oily compounds on the surface of the fibers. After ginning to separate the cellulose-based fibers from other plant materials, such as seeds, the raw fiber material may include substantial quantities of impurities (particulates, bits of plant matter, etc.) trapped within the fibrous matrices and/or adhered to the waxes and oils. In order to make raw natural fibers commercially acceptable for most uses, the fibers may first be processed in several steps to remove the impurities. Such processes, however, may also remove the natural waxes and oils and render the natural fiber more hydrophilic. Processed, cellulose-based fibers may exhibit an increased tendency to form hydrogen bonds with polar liquid bodily exudates, such as urine, for example. Polar fluids may form strong hydrogen bonds with the processed hydrophilic cellulose-based fibers, which may cause the cellulose-based fibers to swell. The hydrogen bonds may be stronger than the capillary forces drawing polar fluids into the underlying acquisition layer and/or absorbent core of an absorbent article, thereby preventing, or at least inhibiting, the rapid transfer of the polar away from the body of the wearer in an absorbent article context.

Without wishing to be bound by theory, it is believed that majority parts of nonwoven having a first contact angle no less than 100° is hydrophobic which may minimize hydrogen bond formation between cellulose-based fibers and polar liquid.

The second surface can tolerate a certain hydrophilicity. However, if the second area (or the second surface) is too hydrophilic, a high level of hydrogen bonds may be formed between cellulose-based fibers and fluid, so that the fluid is trapped among fibers. It may cause a high rewet in the nonwoven. Hydrophobicity difference between the first surface and the second surface which is less hydrophobic than the first surface or slightly hydrophilic may generate hydrophobic gradient from the first surface to the second surface which drives the fluid, especially small amount of fluid which has insufficient gravity, to flow through.

With a hydrophobic first surface, a resistance of the liquid penetrating from the first surface to the second surface of the nonwoven becomes very high which may require a hydrophobic gradient to drive the liquid to penetrate the nonwoven in a thickness direction. Without wishing to be bound by theory, it is believed that if the contact angle difference is too low such as lower than about 15°, hydrophobic gradient may be insufficient to drive the fluid to flow smoothly from the first surface to the second surface of the nonwoven. If the contact angle difference is too high such as higher than about 45°, it may negatively affect dryness of nonwoven as the relatively hydrophilic second surface would generate a force to drive the fluid to flow downwards, while the relatively hydrophobic first surface would generate a force to push the fluid upwards. Therefore, a tension in fluid flow is generated inside the nonwoven. If the tension is higher than the fluid cohesion force, the continuous fluid flow would break into disperse phases. Then, the disperse phases may be trapped within the nonwoven or a fluid may smear on the first surface of the nonwoven, and not flow toward the second surface of the nonwoven.

In order to ensure that the polar fluid(s) contacting a first surface, a wearer-facing surface, of a nonwoven will move suitably rapidly via capillary action in a z-direction toward an opposite second surface, a garment-facing surface, of the nonwoven, where the polar fluids can be drawn into the underlying absorbent layer(s), a first surface of the nonwoven may be treated with a first treatment composition to render the first surface to have a contact angle no less than about 100°, as measured Contact Angle Test disclosed herein.

The first treatment composition may reduce or prevent polar fluid contact with cellulose-based fibers in the nonwoven while still allowing the polar fluid to pass through the fiber matrix of the nonwoven. The first treatment composition may be applied on at least one area of, or an entire first surface of the nonwoven. The first surface may be a wearer-facing surface when the nonwoven is used as a topsheet in an absorbent article.

The first treatment composition may comprise a naturally-derived or synthetic hydrocarbon, triglyceride, diglyceride, ester wax or silicone.

In order to ensure that the polar fluid(s) contacting a first surface, a wearer-facing surface, of a nonwoven will move suitably rapidly via capillary action in a z-direction toward an opposite second surface, a garment-facing surface, of the nonwoven, where the polar fluids can be drawn into the underlying absorbent layer(s), a second surface of the nonwoven may be treated with a second treatment composition to render the second surface to have a second contact angle no less than about 82°, as measured Contact Angle Test disclosed herein which is lower than the first contact angle in the range of about 15°to about 45°. The second treatment composition may reduce or prevent polar fluid contact with the cellulose-based fibers of the nonwoven and allow the polar fluid to move toward layers underneath a nonwoven topsheet when the nonwoven is used as a topsheet in an absorbent article. The second treatment composition may be applied on at least an area of, or an entire second surface of the nonwoven. The second surface may be a garment-facing surface when the nonwoven is used as a topsheet in an absorbent article.

The second treatment composition may comprise a naturally-derived or synthetic hydrocarbon, triglyceride, diglyceride, ester wax or silicone. The second treatment composition may comprise a less hydrophobic compound such as fatty amides. The second treatment composition may be a semi-hydrophilic composition comprising a wax ester and a polyglyceryl emulsifier.

The wax composition of the present disclosure may be insoluble or at least not readily soluble in water or other polar fluids, including, for example, bodily exudates such as urine. The wax composition may reduce or prevent fluid contact with the fibers comprising the topsheet by, for example, forming a physical barrier by treating at least a portion of, or all of, the fibers that comprise the topsheet. As discussed above, the wax composition of the present disclosure may comprise a wax ester. The wax ester may comprise an ester of a fatty acid and a fatty alcohol. The fatty acid component of the wax ester may be a long chain fatty acid or a very long chain fatty acid, comprising between about 14 to about 26 carbon atoms. The fatty alcohol of the wax ester may be a long chain or a very long chain fatty alcohol, comprising between about 14 to about 26 carbon atoms. The wax ester may have a molecular chain length of between about 28 and about 48 carbon atoms, or between about 36 and about 46, or between about 40 and about 44 carbon atoms. The wax ester may comprise saturated fatty acids and fatty alcohols, monounsaturated fatty acids and fatty alcohols, polyunsaturated fatty acids and fatty alcohols, and combinations thereof. The wax ester may have a degree of unsaturation. The degree of unsaturation of the wax ester may be measured by the Iodine Value Test, as disclosed herein. The wax ester may have an iodine value of between about 35 g/100g and about 50 g/100g, between about 38 g/100g and about 48 g/100g, or between about 40 g/100g and about 44 g/100g, according to the Iodine Value Test disclosed herein. Without wishing to be bound by theory, it is believed that a wax ester having a degree of unsaturation as described above may assist in the formation of a semi-hydrophilic wax composition that remains pliable upon application to a topsheet and/or to the fibers of a topsheet. Wax esters having an iodine value lower than that described above may tend to harden and become resistant to flexing or bending, which is not desirable. In the context of an absorbent article, such pliability may be beneficial because an absorbent article may be bent and flexed during the course of use and wear, requiring the wax composition to bend and flex along with the article. The application of a wax composition comprising a wax ester with a low iodine value (corresponding to a low degree of unsaturation) to the topsheet of an absorbent article may result in the wax composition cracking and flaking off of the topsheet during use. This may cause the cellulose-based fibers to be exposed to polar fluids and to swell when the polar fluids contact the cellulose-based fibers.
The second treatment composition is the same as the first treatment composition. When the second treatment composition is the same as the first treatment composition, the level of the second treatment composition on the second area may be lower than the level of the first treatment composition on the first area.

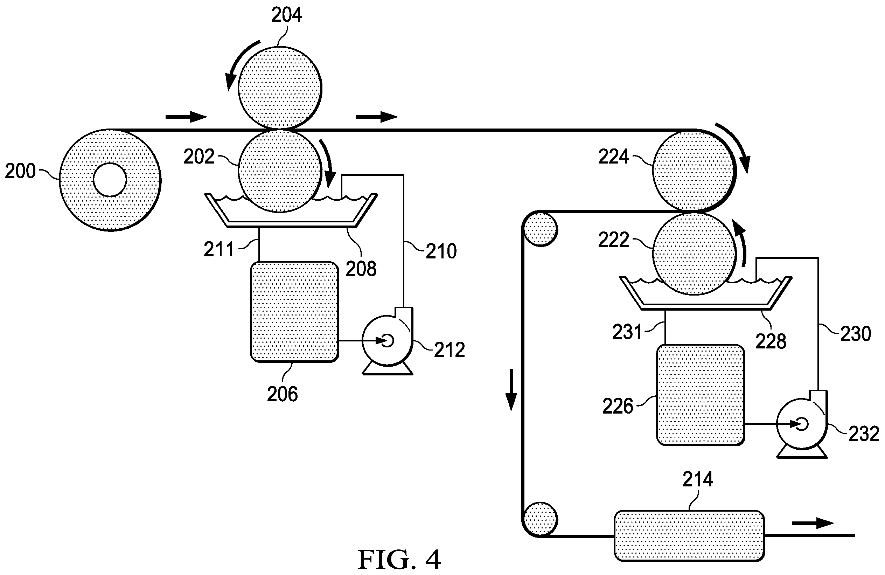

The first and/or the second treatment composition may be applied using an appropriate known method such as a kiss roll coating process illustrated in FIG. 4 and a spray method.

Nonwoven disclosed herein may comprise a plurality of three dimensional elements such as protrusion, recesses, apertures and any combination thereof, so that the nonwoven has a three-dimensional structure.

Fibers making the nonwoven substrate may be processed to be suitably soft-feeling against the skin.

The nonwoven disclosed herein may comprise a spunlace web.

In some embodiment the nonwoven disclosed herein comprises cellulose-based fibers selected from the group consisting of cotton fibers, regenerated cellulose-based fibers, and combinations thereof. The nonwoven may comprise more than 50%, more than 60%, more than 70%, more than 80%, or more than 90% cellulose-based fibers by weight of the first nonwoven layer. In some embodiments, fibers forming the nonwoven comprises 100% cellulose-based fibers. In one embodiment, fibers forming the nonwoven substrate comprises 100% cotton fibers.

The nonwoven disclosed herein may optionally comprise synthetic fibers. Synthetic fibers for nonwovens discussed herein may comprise multi-constituent fibers, such as bi-component fibers or tri-component fibers, mono-component fibers, and/or other fiber types, for example. Multi-constituent fibers, as used herein, means fibers comprising more than one chemical species or material (i.e., multi-component fibers). Bi-component fibers are merely an example of multi-constituent fibers. The fibers may have round, triangular, tri-lobal, or otherwise shaped cross-sections, for example. In a continuous fiber or carded fiber context, it may be desirable to have fibers comprising more than one polymer component, such as bi-component fibers. Often, these two polymer components have different melting temperatures, viscosities, glass transition temperatures, and/or crystallization rates. As the multi-component fibers cool after formation, a first polymer component may solidify and/or shrink at a faster rate than a second polymer component, while the second polymer component may have sufficient rigidity to resist compression along a longitudinal fiber axis. The continuous fibers may then deform and curl up when strain on the fiber is relieved, thereby causing what is known as "crimp" in the fibers. Crimp of the fibers may aid in the softness and loft of a nonwoven or topsheet, which is consumer desirable.

Bi-component fibers may comprise, for example, a first polymer component having a first melting temperature and a second polymer component having a second melting temperature. A first polymer component may comprise polypropylene and a second polymer component may comprise polyethylene, for example. As another example, a first polymer component may comprise polyethylene and a second polymer component may comprise polyethylene terephthalate. As yet another example, a first polymer component may comprise polyethylene and a second polymer component may comprise polylactic acid. If tri-component fibers are used, at least one polymer component may have a different melting temperature (in the ranges specified above) than a melting temperature of at least one of the other two polymer components.

The fibers for the nonwoven disclosed herein may comprise petroleum sourced resins, recycled resins, or bio-sourced resins, such as polylactic acid from Nature Works and polyethylene from Braskem. The fibers may be or may comprise continuous fibers, such as spunbond fibers and melt-blown fibers. Staple fibers, either petroleum-sourced or bio-sourced, such as cotton, cellulous, and/or regenerated cellulous may also be included in a nonwoven. The multi-constituent fibers, such as bi-component fibers, may comprise sheath/core, side By-side, islands in the sea, and/or eccentric configurations or may have other configurations.

Nonwoven disclosed herein may have a basis weight of about 20gsm- about 100gsm, or about 25gsm- about 80gsm, or about 30gsm- about 50gms. When nonwoven has a basis weight too low, a capillary gradient may not be generated effectively.

Nonwoven disclosed herein may be a laminate nonwoven comprising a layer comprising the nonwoven disclosed herein. The laminate nonwoven may further comprise a second nonwoven layer comprising synthetic fibers.

The nonwoven disclosed herein may have a rewet lower than about 70mg, or lower than about 65mg, or lower than about 60mg, as measured by Rewet Test disclosed herein.

Nonwoven disclosed herein may comprise a single layer. It may comprise two or more layers, which may form a unitary structure or may remain as discrete layers which may be attached at least partially to each other by, for example, thermal bonding, adhesive bonding or a combination thereof. A unitary structure herein intends to mean that although it may be formed by several sub-layers that have distinct properties and/or compositions from one another, they are somehow intermixed at the boundary region so that, instead of a definite boundary between sub-layers, it would be possible to identify a region where the different sub-layers transition one into the other. Such a unitary structure is typically built by forming the various sub-layers one on top of the other in a continuous manner, for example using air laid or wet laid deposition. Typically, there is no adhesive used between the sub-layers of the unitary material. However, in some cases, adhesives and/or binders can be present although typically in a lower amount that in multilayer materials formed by separate layers.

The nonwoven substrate may be formed by any suitable process known in nonwoven industry. Nonwoven web may be formed by suitable processes for example, carding, airlaying, and wetlaying. In another example, nonwoven web may be formed by a co-forming process in which cellulose-based fibers of finite lengths are blended or mixed with streams of polymeric resin-based spun fibers of generally longer length than the cellulose-based fibers, and laid down on a forming belt to form a web. The web may be processed to consolidate the fibers and entangle them in the z-direction by processes that may include, for example, calendaring, needle punching, and hydroentanglement with water jets (also known as spunlace).

### Method for Nonwoven Treatment with Treatment Composition

### Kiss roll coating method

Nonwoven where both sides of the nonwoven have different contact angles may be produced generally by the process schematically illustrated in Fig. 4. First, to coat the first surface of the nonwoven with a first treatment composition. The first treatment composition may be prepared and stored in a mixing tank 206. The mixing tank 206 may comprise an agitation system capable of maintaining the first treatment composition in water, wherein the first treatment composition droplets have a mean droplet size of less than about 25 microns. The mixing tank 206 may be in fluid communication with a soaking tank 208 through circulation pipes 210, 211 and a circulation pump 212, for example. The circulation pump may be a high-shear pump capable of maintaining the microemulsion of treatment composition in water. The mixing tank 206 and soaking tank 208 may be maintained at a temperature range of about 50°C to about 90°C, or about 60°C to about 80°C. A nonwoven web 200 from a spool or otherwise is fed between a mesh roll 202 and a compression roll 204. The mesh roll 202 may comprise a plurality of small depressions or pockets engraved into the surface of the mesh roll 202. The mesh roll 202 may be heated to a temperature of between about 60°C and about 90°C. The mesh roll 202 may be disposed so as to be at least partially submerged in the first treatment composition in the soaking tank 206, so that, as the mesh roll 202 passes through the first treatment composition, an amount of the first treatment composition may be picked up and carried in the small depressions or pockets in the surface of the mesh roll 202. The mesh roll 202 may be driven in a first direction (as indicated by the arrow) to match the feed direction of the nonwoven web 200. The compression roll 204 may comprise a resilient or compressible surface, such as, rubber. As the nonwoven web is fed between the mesh roll 202 and the compression roll 204, the compression roll 204 may bring the nonwoven web 200 into contact with the mesh roll 202, and the first treatment composition contained within the small depressions or pockets engraved into the surface of the mesh roll 202. Upon contact of the nonwoven web 200 with the first treatment composition, the treatment composition may adsorb to the first surface of the nonwoven web 200, or to diffuse from the first surface of the nonwoven web 200 to its second surface to form a gradient of the first treatment composition along the thickness of the nonwoven web 200.

Then, if needed, a second surface of the nonwoven is coated with a second treatment composition. A second treatment composition may be prepared and stored in a mixing tank 226. The mixing tank 226 may comprise an agitation system capable of maintaining a second treatment composition in water, wherein the second treatment composition droplets have a mean droplet size of less than about 25 microns. The mixing tank 226 may be in fluid communication with a soaking tank 228 through circulation pipes 230, 231 and a circulation pump 232, for example. The circulation pump may be a high-shear pump capable of maintaining the microemulsion of treatment composition in water. The mixing tank 226 and soaking tank 228 may be maintained at a temperature range of about 50°C to about 90°C, or about 60°C to about 80°C. A nonwoven web 200 from a spool or otherwise is fed between a mesh roll 222 and a compression roll 224. The mesh roll 222 may comprise a plurality of small depressions or pockets engraved into the surface of the mesh roll 222. The mesh roll 222 may be heated to a temperature of between about 60°C and about 90°C. The mesh roll 222 may be disposed so as to be at least partially submerged in the second treatment composition in the soaking tank 228, so that, as the mesh roll 222 passes through the second treatment composition, an amount of the second treatment composition may be picked up and carried in the small depressions or pockets in the surface of the mesh roll 222. The mesh roll 222 may be driven in a first direction (as indicated by the arrow) to match the feed direction of the nonwoven web 200. The compression roll 224 may comprise a resilient or compressible surface, such as, rubber. As the nonwoven web is fed between the mesh roll 222 and the compression roll 224, the compression roll 224 may bring the nonwoven web 200 into contact with the mesh roll 222, and the second treatment composition contained within the small depressions or pockets engraved into the surface of the mesh roll 222. Upon contact of the nonwoven web 200 with the second treatment composition, the treatment composition may adsorb to the second surface of the material web 200. During operation, the pressure between the compression roll 224 and mesh roll 222 may be controlled at the lower limit to minimize the diffusion of the second treatment composition from the second surface of the nonwoven web 200 to its first surface.

The nonwoven web 200 may then fed into a drying oven 214, such as a through-air drying oven, for example. The drying oven may be maintained at a temperature of between about 120°C and about 150°C.

### Spray method

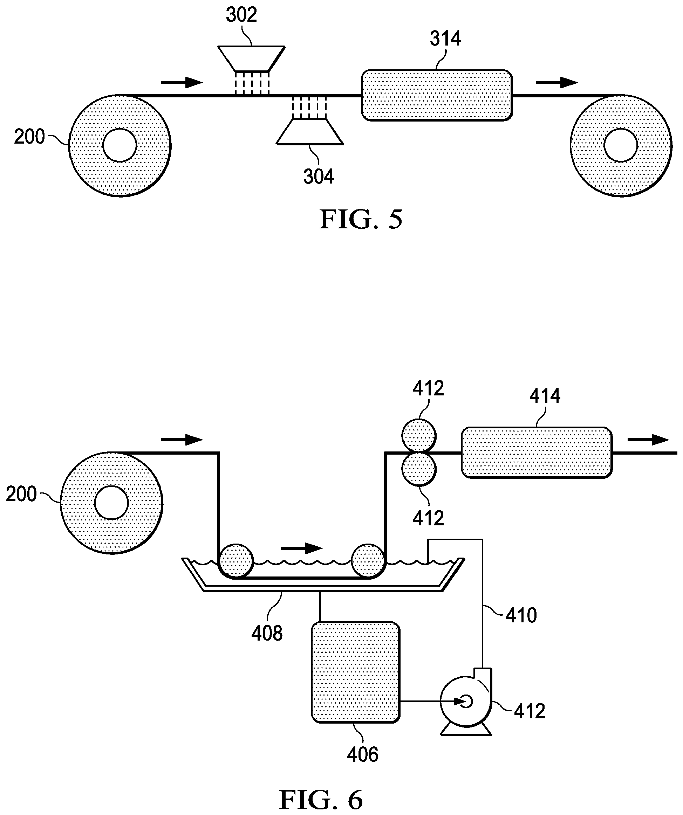

Nonwoven where both sides of the nonwoven have different contact angles may be produced generally by another process schematically illustrated in Fig. 5. First, to coat the first surface of the nonwoven with a first treatment composition. The first treatment composition may be sprayed through the first nozzle 302 towards the first surface of the nonwoven web 200. The distance between the first nozzle 302 and the first surface of the nonwoven web 200 may be maintained in a range of about 10cm to about 40cm. About 90% of the droplet diameters generated by the first nozzle 302 is less than 20 µm. Upon contact of the nonwoven web 200 with the first treatment composition, the first treatment composition may be adsorbed to the first surface of the nonwoven web 200, and diffuse from the first surface of the nonwoven web 200 toward a second surface thereof to form a gradient of the first treatment composition along the thickness of the nonwoven web 200.

Then, if needed, a second surface of the nonwoven is coated with a second treatment composition. The second treatment composition may be sprayed through the optional second nozzle 304 towards the second surface of the nonwoven web 200. The distance between the second nozzle 304 and the second surface of the nonwoven web 200 may be maintained in a range of about 10cm to about 40cm. About 90% of the droplet diameters generated by the second nozzle 304 is less than 20 µm. Upon contact of the nonwoven web 200 with the second treatment composition, the second treatment composition may be adsorbed to the second surface of the material web 200. The dosing amount of the second nozzle 304 to the second surface may be controlled at a low amount to minimize the diffusion of the second treatment composition from the second surface of the nonwoven web 200 toward the first surface.

The nonwoven web 200 may then fed into a drying oven 314, such as a through-air drying oven, for example. The drying oven may be maintained at a temperature of between about 120°C and about 150°C.

### Soaking method

Nonwoven where both sides of the nonwoven have identical contact angles may be produced generally by the process schematically illustrated in Fig. 6. A treatment composition may be prepared and stored in a tank 408. The tank 408 may comprise an agitation system capable of maintaining the treatment composition in water, wherein the treatment composition droplets have a mean droplet size of less than about 25 microns. The tank 408 may be maintained in a temperature range of about 50°C to about 90°C, or about 60°C to about 80°C. A nonwoven web 200 from a spool or otherwise is fed into the tank 408, and the residence time of the nonwoven web 200 in the tank 408 is controlled to be in the range of about 2 seconds to 10 seconds. Upon contact of the nonwoven web 200 with the treatment composition, the treatment composition may be adsorbed to the nonwoven web 200.

When the nonwoven web 200 runs out of the tank 408, the nonwoven web 200 is squeezed by a pair of nipping rolls 412. The nipping rolls 412 may comprise a resilient or compressible surface, such as, rubber.

The nonwoven web 200 may then fed into a drying oven 414, such as a through-air drying oven, for example. The drying oven may be maintained at a temperature of between about 120°C and about 150°C.

### MEASUREMENT

### Iodine Value Test:

The Iodine Value of a composition is determined using the American Oil Chemists' Society (AOCS) standard test method Cd 1d-92.

### Contact Angle Test

The Contact Angle Test is used to measure the contact angle made by highly purified water (18 M Ω-cm) in contact with constituent fibers of a nonwoven using a DataPhysics OCA 50 controlled via TP50 (DataPhysics Instruments GmbH, Filderstadt, Germany) or equivalent. All measurements are performed at an ambient temperature of 24 ± 2°C.

Raw material nonwoven is analyzed as received without compression, and nonwoven in a finished article is excised as received in the article. If a topsheet is available in its raw material form, six representative nonwoven specimen areas measuring approximately 5 cm ± 0.5 cm in length and 5 cm ± 0.5 cm wide are excised from a nonwoven of interest are prepared, and each is analyzed sequentially. Otherwise, a topsheet specimen is removed from an absorbent article, centered at the intersection of the longitudinal and lateral centerlines of the absorbent article. For the purposes of removing the topsheet from the absorbent article, a razorblade is used to excise the topsheet from the underlying layers of the absorbent article. The topsheet is carefully removed such that its longitudinal and lateral extension are maintained. A cryogenic spray (such as Cyto-Freeze, Control Company, Houston, TX) can be used to remove the topsheet specimen from the underlying layers, if necessary. In cases where the topsheet comprises multiple layers, only the top-most (wearer-facing) layer of the multi-layer topsheet serves as the specimen. A cryogenic spray can be used to remove the top-most layer specimen from other layers of the topsheet, if necessary. Six flat areas are selected to test contact angles.

2 µL of highly purified water are placed on the surface to be tested of nonwoven specimen (or each flat area selected in case topsheet specimen), and the camera (operating at 20 frames/second) is used to capture the droplet shape on the nonwoven surface. Time zero is defined as the first frame in which the droplet is observed to touch the nonwoven surface, and the frame at 5.0 seconds is captured and further analyzed using the instrument's software to determine the contact angle between the droplet and a fiber on the nonwoven surface. In this analysis, the baseline of the fiber is identified manually, and the Laplace-Young fitting mode is used to determine the contact angle. Six like specimens are analyzed and the arithmetic mean of the six individual contact angle results is calculated and reported to the nearest integer° as the contact angle of the nonwoven.

### Rewet Test

Rewet Test is used to determine rewet value for nonwoven rewet on two specimens. A nonwoven to test is placed on 5 layers of filter paper (Ahlstrom grade 632 filter paper, Ahlstrom-Munksio, Helsinki, Finland, or equivalent) in such a way that the first surface of the nonwoven faces up. The nonwoven and filter papers are placed on a plastic plate, and put under the discharge position of LISTER instrument (Lenzing Instrument GmbH & Co., KG), or equivalent to apply fluid in a pre-determined dose. The electrode tester of LISTER is place on top of the nonwoven. 5 ml of 0.9% NaCl solution is discharged through LISTER, and when the timer of the LISTER stops, remove the plastic plate together with the nonwoven and filter papers within 30 seconds to start the rewet test.

For each specimen analyzed, first one layer of filter paper (Ahlstrom grade 632 filter paper, Ahlstrom-Munksio, Helsinki, Finland, or equivalent) is pre-weighed, and its mass is recorded as M1 to the nearest 0.1 mg.

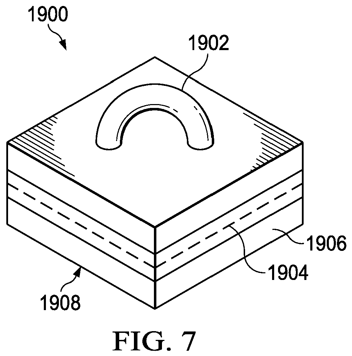

The pre-weighed single layer of filter paper, defined as the pick-up paper, is placed on top of the first surface of the nonwoven. A weight is placed on top of the pick-up paper and topsheet for 120 seconds. Fig. 7 is a schematic illustration of the weight 1900. Referring to Fig. 7, the weight 1900 comprises a weight 1902 with a 10 cm x 10 cm stainless steel base and a handle, with a total mass of 4000 g ± 20 g. Tape 1904 is placed on the underside of the base of the weight. Polyurethane foam rubber 1906 with dimensions of 10 cm x 10 cm x 2 cm is affixed to the tape 1904. A polyethylene film 1908 with dimensions 10 cm x 10 cm x 25 µm is then attached to the underside of the polyurethane foam rubber 1906. After 120 seconds, the pick-up paper is removed from the nonwoven and weighed again, the mass recorded to the nearest 0.1 mg as M2. The rewet mass for the specimen is defines as M2-M1. The arithmetic mean of the rewet masses of two like specimen replicates is defines as the rewet and is reported to the nearest 0.1 mg.

### EXAMPLES

### Example 1: Preparation of Nonwovens

Using 35gsm 100% cotton spunlace nonwoven (Xiamen Yanjan New Material Co., Ltd., China), coated nonwovens were prepared according to Table 1 below.

**Table 1**

| Nonwoven | Treatment |
|---|---|
| A | No treatment |
| B | Spray 5% L689*¹ emulsion via two discharges from a nozzle on the first surface of nonwoven. |
| | No treatment on the second surface of nonwoven |
| C | Spray 5% L689 emulsion via 4 discharges from a nozzle on the first surface of nonwoven. |
| | No treatment on the second surface of nonwoven |
| D | Spray 10% L689 emulsion via 4 discharges from a nozzle on the first surface of nonwoven. |
| | No treatment on the second surface of nonwoven |
| E | Spray 3% Jojoba semi-hydrophilic emulsion *² via 3 discharges from a nozzle on the second surface of nonwoven. |
| | Then, Spray 10% Stantex S 6787N*³ emulsion via two discharges from a nozzle on the first surface of nonwoven. |
| F | Spray 10% Stantex S 6787N emulsion via two discharges from a nozzle on the first surface of nonwoven. |
| | Then, spray 5% L689 emulsion via two discharges from a nozzle on the second surface of nonwoven. |
| G | Spray 10% Stantex S 6787N emulsion via two discharges from a nozzle on the first surface of nonwoven. |
| | Then, spray 7% L689 emulsion via two discharges from a nozzle on the second surface of nonwoven. |
| H | Spray 2.5% Jojoba semi-hydrophilic emulsion via 3 discharges from a nozzle on the second surface of nonwoven. |
| | Then, spray10% PP-14854*⁴ emulsion via 3 discharges from a nozzle on the first surface of nonwoven. |
| I | Spray 3% Jojoba semi-hydrophilic emulsion via 3 discharges from a nozzle on the second surface of nonwoven. |
| | Then, spray 10% PP-14854 emulsion via 3 discharges from a nozzle on the first surface of nonwoven. |
| J | Spray 2.5% Jojoba semi-hydrophilic emulsion via 3 discharges from a nozzle on the second surface of nonwoven. |
| | Then, spray 1% L689 emulsion spray via one discharge from a nozzle on the first surface of nonwoven |
| K | Spray 6% active L689 emulsion via two discharges from a nozzle on the first surface of nonwoven. |
| | Then, spray 4% active L689 emulsion via two discharges from a nozzle on the second surface of nonwoven. |
| L | Spray 1.5% Jojoba semi-hydrophilic emulsion via two discharges from a nozzle on the second surface of nonwoven. |
| | Then, spray 5% Stantex S 6787N emulsion via two discharges from a nozzle on the first surface of nonwoven. |
| M | Spray 20% active L689 emulsion via one discharge from a nozzle on the first surface of nonwoven. |
| | No treatment on the second surface |
| N | Spray 1.8% Jojoba semi-hydrophilic emulsion via two discharges from a nozzle on the second surface of nonwoven. |
| | Then, spray 5.5% Stantex S 6787N emulsion via one discharge from a nozzle on the first surface of nonwoven. |
| O | Soak nonwoven in 0.2% active L689 emulsion. |
| P | Soak nonwoven in 0.5% active L689 emulsion. |
| Q | Soak nonwoven in 2% active L689 emulsion. |

| | |
|---|---|
| L689^{*1}: Goulston Technologies, Inc., USA Jojoba semi-hydrophilic emulsion^{*2}: Floraesters 60 (Floratech, USA), and PolyAquol 2W (Innovacos Corp, USA) in the ratio of 2:1 by weight. Stantex S 6787N^{*3}: Pulcra Chemicals GmbH, Germany PP-14854^{*4}: Goulston Technologies, Inc., USA | |

### Example 2: Nonwoven Characteristics

Contact angles of both surfaces of each nonwoven prepared in Example 1 were measured according to Contact Angle Test, and rewet of each nonwoven in Example 1 was measured according to Rewet Test disclosed herein. Table 2 below includes measurement results.

**Table 2**

| Nonwoven | Total treatment level | Contact angle on the 1st surface | Contact angle on the 2^{nd} surface | Contact angle difference | Rewet (mg) |
|---|---|---|---|---|---|
| A | 0.00% | 0 | 0 | 0 | 309.9 |
| B | 0.43% | 108.0 | 85.0 | 23.0 | 45.6 |
| C | 1.55% | 128.4 | 110.3 | 18.1 | 44.5 |
| D | 2.80% | 130.6 | 93.8 | 36.8 | 45.6 |
| E | 2.72% | 129.4 | 85.2 | 44.2 | 40.7 |
| F | 2.58% | 131.6 | 115.1 | 16.5 | 41.4 |
| G | 2.70% | 131.4 | 120.7 | 10.7 | 79.7 |
| H | 2.23% | 137.9 | 81.7 | 56.2 | 117.3 |
| I | 2.87% | 137.1 | 88.2 | 48.9 | 109.0 |
| J | 1.80% | 93.2 | 82.1 | 11.1 | 93.2 |
| K | 1.63% | 101.3 | 94.9 | 6.4 | 81.8 |
| L | 1.75% | 101.0 | 71.8 | 29.2 | 114.3 |
| M | 2.13% | 121.2 | 0 | 121.2 | 191.7 |
| N | 2.77% | 109.0 | 81.3 | 27.7 | 99.1 |
| O | 0.49% | 93.7 | 93.3 | 0.4 | 138.8 |
| P | 1.54% | 103.5 | 103.4 | 0.1 | 79.5 |
| Q | 6.96% | 137.1 | 137.2 | 0.1 | 75.0 |

Nonwovens B-F have first surfaces having a first contact angle no less than about 100°, and second surfaces having a second contact angle no less than about 82°. The second contact angle is smaller than the first contact angle in the range of about 15° to about 45°. Nonwovens B-F have much lower rewet values in comparison with other nonwovens, Nonwovens A and G-Q.

## Claims

1. An absorbent article (20) comprising a wearer-facing surface and an opposite garment-facing surface, the absorbent article (20) further comprising a liquid pervious topsheet (24), a liquid impervious backsheet (26), and an absorbent core (28) disposed between the topsheet (24) and the backsheet (26),
wherein the topsheet (24) comprises a nonwoven substrate comprising cellulose-based fibers, a first surface (110) and an opposite second surface (120), the first surface (110) of the nonwoven substrate forming at least part of the wearer-facing surface
wherein the first surface (110) comprises a first area having a first contact angle no less than 100°, as measured by Contact Angle Test, and the second surface (120) comprises a second area having a second contact angle no less than 82°, as measured by Contact Angle Test, the second contact angle being smaller than the first contact angle, and
wherein a difference between the first contact angle and the second contact angle is in the range of 15° to 45°,
wherein the first surface (110) comprises a first treatment composition, and
wherein the second surface (120) comprises a second treatment composition which is the same as the first treatment composition.

2. The absorbent article (20) according to claim 1, wherein the cellulosed-based fibers are fibers selected from the group consisting of cotton fibers, regenerated cellulose fibers and combinations thereof.

3. The absorbent article (20) according to any of the preceding claims, wherein the first contact angle is no greater than 145°.

4. The absorbent article (20) according to any of the preceding claims, wherein the nonwoven substrate has a basis weight no less than 30gsm.

5. The absorbent article (20) according to any of the preceding claims, wherein the nonwoven substrate comprises a plurality of three-dimensional elements.

6. The absorbent article (20) according to any of the preceding claims, wherein the nonwoven substrate comprises natural fibers.

7. The absorbent article (20) according to any of the preceding claims, wherein the nonwoven substrate comprises spunlace nonwoven.

8. The absorbent article (20) according to any of the preceding claims, wherein the nonwoven substrate has a rewet lower than 70mg, as measured by Rewet Test disclosed herein.

9. The absorbent article (20) according to any of the preceding claims, wherein the topsheet (24) has a rewet lower than 70mg, as measured by Rewet Test disclosed herein.

10. The absorbent article (20) according to any of the preceding claims, wherein the topsheet (24) further comprises a second layer positioned underneath the nonwoven substrate.

11. The absorbent article (20) according to claim 10, wherein the second layer comprises synthetic fibers.

12. The absorbent article (20) according to any of the preceding claims, wherein the first surface (110) comprises a plurality of first areas having a first contact angle.

13. The absorbent article (20) according to any of the preceding claims, wherein the absorbent core (28) comprises an absorbent material comprising superabsorbent polymers at least 80%by weight of the absorbent material.

## Patentansprüche

1. Absorptionsartikel (20), umfassend eine trägerseitige Oberfläche und eine entgegengesetzte, bekleidungsseitige Oberfläche, der Absorptionsartikel (20) ferner umfassend eine flüssigkeitsdurchlässige Oberschicht (24), eine flüssigkeitsundurchlässige Unterschicht (26) und einen Absorptionskern (28), der zwischen der Oberschicht (24) und der Unterschicht (26) eingerichtet ist,
wobei die Oberschicht (24) ein Vliessubstrat umfasst, umfassend zellulosebasierte Fasern, eine erste Oberfläche (110) und eine entgegengesetzte zweite Oberfläche (120), wobei die erste Oberfläche (110) des Vliessubstrats wenigstens einen Teil der trägerseitigen Oberfläche ausbildet,
wobei die erste Oberfläche (110) einen ersten Bereich umfasst, der einen ersten Kontaktwinkel von nicht weniger als 100° aufweist, gemessen durch einen Kontaktwinkeltest, und die zweite Oberfläche (120) einen zweiten Bereich umfasst, der einen zweiten Kontaktwinkel von nicht weniger als 82° aufweist, gemessen durch einen Kontaktwinkeltest, wobei der zweite Kontaktwinkel kleiner als der erste Kontaktwinkel ist, und
wobei ein Unterschied zwischen dem ersten Kontaktwinkel und dem zweiten Kontaktwinkel in dem Bereich von 15° bis 45° liegt.
wobei die erste Oberfläche (110) eine erste Behandlungszusammensetzung umfasst, und
wobei die zweite Oberfläche (120) eine zweite Behandlungszusammensetzung umfasst, die dieselbe wie die erste Behandlungszusammensetzung ist.

2. Absorptionsartikel (20) nach Anspruch 1, wobei die zellulosebasierten Fasern Fasern sind, die aus der Gruppe ausgewählt sind, bestehend aus Baumwollfasern, regenerierten Zellulosefasern und Kombinationen davon.

3. Absorptionsartikel (20) nach einem der vorstehenden Ansprüche, wobei der erste Kontaktwinkel nicht größer als 145° ist.

4. Absorptionsartikel (20) nach einem der vorstehenden Ansprüche, wobei das Vliessubstrat ein Flächengewicht von nicht weniger als 30 gsm aufweist.

5. Absorptionsartikel (20) nach einem der vorstehenden Ansprüche, wobei das Vliessubstrat eine Vielzahl von dreidimensionalen Elementen umfasst.

6. Absorptionsartikel (20) nach einem der vorstehenden Ansprüche, wobei das Vliessubstrat Naturfasern umfasst.

7. Absorptionsartikel (20) nach einem der vorstehenden Ansprüche, wobei das Vliessubstrat ein Spunlacing-Vlies umfasst.

8. Absorptionsartikel (20) nach einem der vorstehenden Ansprüche, wobei das Vliessubstrat eine Rücknässung von weniger als 70 mg aufweist, gemessen durch den hierin offenbarten Rücknässungstest.

9. Absorptionsartikel (20) nach einem der vorstehenden Ansprüche, wobei die Oberschicht (24) eine Rücknässung von weniger als 70 mg aufweist, gemessen durch den hierin offenbarten Rücknässungstest.

10. Absorptionsartikel (20) nach einem der vorstehenden Ansprüche, wobei die Oberschicht (24) ferner eine zweite Schicht umfasst, die unter dem Vliessubstrat angeordnet ist.

11. Absorptionsartikel (20) nach Anspruch 10, wobei die zweite Schicht synthetische Fasern umfasst.

12. Absorptionsartikel (20) nach einem der vorstehenden Ansprüche, wobei die erste Oberfläche (110) eine Vielzahl von ersten Bereichen, die einen ersten Kontaktwinkel aufweisen, umfasst.

13. Absorptionsartikel (20) nach einem der vorstehenden Ansprüche, wobei der Absorptionskern (28) ein Absorptionsmaterial umfasst, umfassend zu wenigstens 80 Gew.-% des Absorptionsmaterials Superabsorber-Polymere.

## Revendications

1. Article absorbant (20) comprenant une surface faisant face au porteur et une surface opposée faisant face au vêtement, l'article absorbant (20) comprenant en outre une feuille de dessus (24) perméable aux liquides, une feuille de fond (26) imperméable aux liquides et une âme absorbante (28) disposée entre la feuille de dessus (24) et la feuille de fond (26),
dans lequel la feuille de dessus (24) comprend un substrat non tissé comprenant des fibres à base de cellulose, une première surface (110) et une seconde surface (120) opposée, la première surface (110) du substrat non tissé formant au moins une partie de la surface faisant face au porteur
dans lequel la première surface (110) comprend une première zone ayant un premier angle de contact d'au moins 100°, tel que mesuré par un test d'angle de contact, et la seconde surface (120) comprend une seconde zone ayant un second angle de contact d'au moins 82°, tel que mesuré par un test d'angle de contact, le second angle de contact étant plus petit que le premier angle de contact, et
dans lequel une différence entre le premier angle de contact et le second angle de contact est dans la plage de 15° à 45°.
dans lequel la première surface (110) comprend une première composition de traitement, et
dans lequel la seconde surface (120) comprend une seconde composition de traitement qui est la même que la première composition de traitement.

2. Article absorbant (20) selon la revendication 1, dans lequel les fibres à base de cellulose sont des fibres choisies dans le groupe constitué par fibres de coton, fibres de cellulose régénérées et combinaisons de celles-ci.

3. Article absorbant (20) selon l'une quelconque des revendications précédentes, dans lequel le premier angle de contact n'est pas supérieur à 145°.

4. Article absorbant (20) selon l'une quelconque des revendications précédentes, dans lequel le substrat non tissé a une masse surfacique d'au moins 30 g/ₘ².

5. Article absorbant (20) selon l'une quelconque des revendications précédentes, dans lequel le substrat non tissé comprend une pluralité d'éléments tridimensionnels.

6. Article absorbant (20) selon l'une quelconque des revendications précédentes, dans lequel le substrat non tissé comprend des fibres naturelles.

7. Article absorbant (20) selon l'une quelconque des revendications précédentes, dans lequel le substrat non tissé comprend un non-tissé lacé par filage.

8. Article absorbant (20) selon l'une quelconque des revendications précédentes, dans lequel le substrat non tissé a un remouillage inférieur à 70 mg, tel que mesuré par le test de remouillage décrit ici.

9. Article absorbant (20) selon l'une quelconque des revendications précédentes, dans lequel la feuille de dessus (24) a un remouillage inférieur à 70 mg, tel que mesuré par le test de remouillage décrit ici.

10. Article absorbant (20) selon l'une quelconque des revendications précédentes, dans lequel la feuille de dessus (24) comprend en outre une seconde couche positionnée en dessous du substrat non tissé.

11. Article absorbant (20) selon la revendication 10, dans lequel la seconde couche comprend des fibres synthétiques.

12. Article absorbant (20) selon l'une quelconque des revendications précédentes, dans lequel la première surface (110) comprend une pluralité de premières zones ayant un premier angle de contact.

13. Article absorbant (20) selon l'une quelconque des revendications précédentes, dans lequel l'âme absorbante (28) comprend un matériau absorbant comprenant des polymères superabsorbants à raison d'au moins 80 % en poids du matériau absorbant.
